# EUROPÄISCHE PATENTSCHRIFT

(11) **EP 1 121 109 B1**
(45) Veröffentlichungstag und Bekanntmachung des Hinweises auf die Patenterteilung: **31.05.2006**
(21) Anmeldenummer: 99953856.4
(22) Anmeldetag: 15.10.1999
(51) Int. Cl.: A61K 31/135, A61K 9/20, A61P 25/04, A61K 9/52

(54) **Zweiphasige Zusammensetzung mit Tramadol**
Biphasic tramadol preparation
Formulation a liberation biphasique contenant du tramadol

(30) Priorität: 15.10.1998 DE 29818454 U
(43) Veröffentlichungstag der Anmeldung: 08.08.2001
(73) Patentinhaber: Euroceltique S.A., Luxembourg (LU)
(72) Erfinder: WIMMER, Walter, D-65549 Limburg (DE); BRÖGMANN, Bianca, D-65552 Limburg-Eschhofen (DE); HAHN, Udo, D-56412 Nentershausen (DE); SPITZLEY, Christof, D-65627 Elbtal-Heuchelheim (DE)
(74) Vertreter: Maiwald Patentanwalts GmbH
(86) Internationale Anmeldenummer: PCT/EP1999/007842
(87) Internationale Veröffentlichungsnummer: WO 2000/021520

(56) Entgegenhaltungen:
- EP-A- 0 624 366
- EP-A- 0 631 781
- EP-A- 0 642 788
- EP-A- 0 864 325
- WO-A-95/25506
- WO-A-97/32573
- US-A- 5 645 858
- In vitro Freisetzungsraten von DHC 60 mg Mundipharm Retardtabletten, MST 60 mg und 100 mg Mundipharm Retardtabletten ; eingereicht durch Grünenthal GmbH am 31.05.96 als Annex zum Einspruchsschrift im Einspruchsverfahren gegen EP-B-624,366 / EP 94 303 128.

## Beschreibung

Die Erfindung betrifft ein pharmazeutisches Präparat, insbesondere zur oralen Verabreichung, mit wenigstens einem Wirkstoff und mit die Wirkstoff-Freisetzung beeinflussenden Formulierungsbestandteilen, das als Wirkstoff wenigstens ein Opioid-Analgetikum umfaßt, welches anteilig einerseits zur schnellen Freisetzung, andererseits zur verzögerten Freisetzung formuliert ist. Die Erfindung betrifft pharmazeutische Präparate, die als Wirkstoff Tramadol bzw. ein pharmazeutisch verträgliches Salz von Tramadol, speziell Tramadol-Hydrochlorid enthalten.

Sogenannte "mehrphasige" pharmazeutische Präparate, bei denen der Wirkstoffgehalt einerseits zur schnellen, andererseits zur verzögerten (retardierten) Freisetzung formuliert ist, sind seit langem bekannt. Solche Präparate sind auch schon mehrfach für Analgetika beschrieben worden.

Aus EP-A1 0 243 366 ist eine Retardformulierung für Tramadol bekannt, die zu einer gleichmäßigen und lang andauernden Freisetzung des Wirkstoffes über vierundzwanzig Stunden oder mehr geeignet ist.

Aus EP-A1 0 642 788 sind pharmazeutische Präparate in Tablettenform bekannt, die als Wirkstoff ein Tramadolsalz enthalten, das in einer Matrix zur verzögerten Wirkstoff-Freisetzung vorliegt ist. Wesentlicher Bestandteil dieser Matrix sind Celluloseether und / oder Celluloseester, die in einer 2 Gew.-%igen wäßrigen Lösung bei 20°C eine Viskosität zwischen 3000 und 150.000 mPas aufweisen. In der Beschreibung ist darauf hingewiesen, daß der Tablettenkern, der die wirkstoffhaltige Matrix enthält, mit zusätzlichem Wirkstoff umhüllt sein kann, der nicht retardiert ist und also schnell freigesetzt wird. Als Beispiele sind Mehrschicht- und Manteltabletten genannt.

Mehrphasige Tramadol-Präparate sind im Handel erhältlich. Ein Beispiel bildet das Produkt "Tramadolor ID 100" der Hexal AG, das Tramadol einerseits in retardierter, andererseits in schnell freisetzender Formulierung enthält. Die *in vitro* Freisetzungsdaten wie auch die Blutplasmaspiegel-Entwicklung mit der Zeit entsprechen jedoch noch einer relativ langsamen anfänglichen Anflutung, so daß der Eintritt der gewünschten analgetischen Wirkung nach Verabreichung des Präparates nicht immer so schnell erfolgt, wie dies wünschenswert wäre.

Aus der EP-A2 0 864 325 ist kürzlich ein anderes zweiphasiges Präparat bekannt geworden, welches ein Opioid-Analgetikum, beispielsweise Tramadol-Hydrochlorid, in einerseits retardierter, andererseits schnell freisetzender Formulierung enthält. Gemäß dieser Veröffentlichung ist die *in vitro* Freisetzungsrate (bestimmt nach dem in Ph. Eur. beschriebenen Paddeltest) extrem hoch; wenigstens 50 Gew.-% des Wirkstoffes werden aus dem Präparat innerhalb einer Stunde *in vitro* freigesetzt. Die Beispiele der EP-A2 0 864 325 entsprechen sogar teilweise noch sehr viel höheren Freisetzungsraten: Nach zwei Stunden sind etwa 70 Gew.-%, nach vier Stunden etwa 90 Gew.-% des Wirkstoffes freigesetzt, und nach sechs Stunden ist die Wirkstoff-Freisetzung praktisch abgeschlossen.

Das entspricht einem extrem schnellen Aufbau hoher Plasmaspiegel des Wirkstoffes und einer entsprechend sehr schnell einsetzenden analgetischen Wirkung.

Andererseits ist jedoch in EP-A2-0 864 325 angegeben, daß nach einmaliger Applikation, unter Fasten-Bedingungen, noch nach fünf Stunden Blutplasmaspiegel von 90 bis 200 ng/ml, und im bevorzugten Bereich Blutplasmaspiegel von weit über 100 ng/ml gefunden wurden.

Die einmalig applizierte Wirkstoffdosis, der diese Blutplasmaspiegel-Entwicklung entspricht, ist in der Anmeldung nicht angegeben. Sie läßt sich jedoch größenordnungsmäßig daraus erschließen, daß solche hohen Plasmaspiegelwerte nach fünf Stunden, angesichts der extrem schnellen Gesamtfreisetzung des Wirkstoffes aus dem Präparat, nur möglich sind, wenn die Dosierung des Wirkstoffes in der Einheitsdosis sehr hoch ist. Die Angaben in der EP-A2 0 864 325 entsprechen wahrscheinlich einer Einheitsdosis von 200 mg Wirkstoff. Da eine zweimal tägliche Applikation vorgesehen ist, würde bei einem so hohen Wirkstoffanteil in der Einheitsdosis zwar die in der Tramadol-Monographie des Bundesgesundheitsamtes von 1994 vorgegebene maximale Tagesdosis von 400 mg noch nicht überschritten (aber erreicht), jedoch bedeutet dies auch, daß im Zeitraum zwischen dem ersten Erreichen der analgetisch wirksamen Konzentration einerseits, und dem Abfall des Plasmaspiegels unter die analgetisch wirksame Konzentration andererseits, der Patient über Stunden einer sehr hohen Wirkstoffkonzentration ausgesetzt ist, die weit oberhalb dessen liegt, was für die analgetische Wirkung notwendig wäre.

Andererseits ergibt sich aus den Daten dieser Anmeldung auch, daß die Wirkung im Sinne einer Schmerzfreiheit des Patienten nicht zwölf Stunden anhalten kann. Der sehr schnellen Gesamtfreisetzung des Wirkstoffes entspricht auch eine entsprechend kurze Halbwertsdauer W₅₀. Bei zweimal täglicher Verabreichung wird der Patient also stundenlang zu hoch, und nachfolgend mehrere Stunden lang zu gering mit Wirkstoff versorgt.

Es ist angesichts dieses Standes der Technik eine wesentliche Aufgabe der Erfindung, ein pharmazeutisches Präparat für Tramadol der eingangs genannten Art zu schaffen, das einen möglichst schnellen Eintritt der analgetischen Wirkung mit einer möglichst langen Wirkungsdauer verbindet und dabei zwischenzeitige hohe Plasmakonzentrationen des Wirkstoffes vermeidet.

Es ist eine weitere wesentliche Aufgabe der Erfindung, ein solches Präparat der eingangs genannten Art zu schaffen, das bei zweimal bis dreimal täglicher Dosierung eine gleichmäßige und ausreichende analgetische Wirkung über den gesamten 24-Stunden-Zeitraum ermöglicht.

Weitere Aufgaben und Vorteile der Erfindung ergeben sich aus der folgenden Beschreibung.

### Erfindungsgemäß bevorzugte Präparate umfassen als Wirkstoff wenigstens

Tramadol und/oder dessen physiologisch verträgliche Salze sowie Derivate der vorgenannten Wirkstoffe.

Grundsätzlich liegt die erfindungsgemäße Lösung der Aufgabe, gemäß den Merkmalen des unabhängigen Anspruchs, in der Formulierung des pharmazeutischen Präparates derart, daß das im Präparat enthaltene Opioid-Analgetikum tramadol, insbesondere ein Tramadolsalz wie Tramadol-Hydrochlorid, teilweise zur schnellen Freisetzung formuliert ist und teilweise zur verzögerten Freisetzung formuliert ist, und zwar so, daß die *in vitro* Freisetzungsrate aus dem Präparat gemäß dem Paddeltest laut Ph. Eur. im Mittelwert nach einer Stunde schon bei oberhalb 40 Gew.-% Wirkstoff liegt, andererseits aber nach vier Stunden noch nicht 80 Gew.-% Wirkstoff unter gleichen Testbedingungen erreicht, und das Präparat den zur verzögerten Freisetzung formulierten Anteil des Wirkstoffs in einer Retardmatrix enthält, die eine oder mehreren Alkylcellulosen gemeinsam mit einem oder meheren aliphatischen C₁₂-C₃₆ Alkoholem umfasst.

In einer bevorzugten Ausführungsform der Erfindung sind nach vier Stunden *in vitro* schon mehr als 70 Gew.-% des Wirkstoffes freigesetzt. Nach sieben bis zehn Stunden, und besonders bevorzugt etwa nach acht Stunden, sind *in vitro* etwa 90 Gew.-% des Wirkstoffes freigesetzt. Eine vollständige Freisetzung ist vorzugsweise jedoch noch nach vierzehn Stunden, insbesondere noch nach sechzehn Stunden nicht erreicht.

Im Vergleich mit EP-A2 0 864 325 ist der anfängliche Verlauf der Freisetzung, etwa bis zu einer Stunde, ähnlich. Die Freisetzung nach einer Stunde erreicht in jedem Fall 40 Gew.-%, muß aber nicht die im Stand der Technik vorgeschriebenen 50 Gew.% erreichen. Es ist allerdings möglich, und wird auch bevorzugt, wenn die Freisetzung *in vitro,* gemäß dem genannten Paddeltest, im Mittelwert nach einer Stunde etwa bei 50 Gew.-% oder sogar noch darüber liegt.

Das läßt sich durch entsprechende Einstellung der Freisetzungsrate erreichen. Hierzu wird man insbesondere die Art und Menge des Sprengmittels heranziehen, das im schnell freisetzenden Anteil des Präparates eingearbeitet ist. Grundsätzlich läßt sich die Wirkstoffmenge, die innerhalb der ersten Stunde im Mittel beim Paddeltest freigesetzt wird, dadurch steigern, daß der Anteil an Sprengmittel erhöht wird. Erfindungsgemäß geeignete Sprengmittel umfassen vorzugsweise ein Polymer und besonders bevorzugt quer-vernetztes Polyvinylpyrrolidon (PVP).

Besonders im Zeitbereich ab zwei Stunden nach Verabreichung unterscheidet sich die Freisetzungskurve dann sehr deutlich von der Lehre der EP-A2 0 864 325. Die Freisetzung erfolgt, im wesentlichen aus dem Retardanteil des Präparates, sehr viel langsamer, und bis zur Annäherung an die vollständige Freisetzung braucht ein bevorzugtes erfindungsgemäßes Präparat mehr als doppelt so lang (mehr als sechzehn Stunden im Vergleich mit etwa acht Stunden) wie gemäß EP-A2 0 864 325.

Das Erfindungsprinzip läßt sich zusätzlichüber die Wirkstoff-Plasmaspiegel definieren, die nach einmaliger Verabreichung eines erfindungsgemäßen Präparates im Mittel bei einer Mehrzahl von Probanden gemessen werden. Die zeitliche Entwicklung des Wirkstoff-Plasmaspiegels für ein erfindungsgemäßes Präparat ist etwa folgende:

| | | | | | | | |
|---|---|---|---|---|---|---|---|
| Mindestens | 2 ng/ml | Vorzugsweise mindestens | 3 ng/ml | Besonders bevorzugt mindestens | | 4 ng/ml | nach 0.15 h |
| " | 10 ng/ml | " | 13 ng/ml | " | " | 15 ng/ml | nach 0.30 h |
| " | 35 ng/ml | " | 40 ng/ml | " | " | 45 ng/ml | nach 0,50 h |
| " | 55 ng/ml | " | 60 ng/ml | " | " | 65 ng/ml | nach 0,75 h |
| " | 75 ng/ml | " | 80 ng/ml | " | " | 85 ng/ml | nach 1,0 h |
| " | 85 ng/ml | " | 90 ng/ml | " | " | 95 ng/ml | nach 1,25 h |
| " | 100 ng/ml | " | 105 ng/ml | " | " | 110 ng/ml | Nach 1,5 h |

wobei die weitere zeitliche Entwicklung dann folgendem entspricht:

| | |
|---|---|
| Plasmakonzentration | Zeitraum ab Verabreichung |
| zwischen 90 und 120 ng/ml | zwischen 2 h und 4 h; |
| zwischen 60 und 100 ng/ml | zwischen 4 h und 8 h; |
| zwischen 40 und 60 ng/ml | zwischen 8 h und 11 h und |

insbesondere oberhalb 30 ng/ml bis 16 h nach Verabreichung.

Diese Werte beziehen sich auf die einmalige Verabreichung einer Dosis von 100 mg des Analgetikums, Tramadol insbesondere Tramadol-Hydrochlorid. Die Absolutwerte des Plasmaspiegels sind natürlich abhängig von der gegebenen Dosis. Jedoch entspricht die zeitliche Entwicklung des Plasmaspiegels auch bei anderen Einmaldosierungen als 100 mg Analgetikum hinsichtlich ihrer Kurvenform, bei entsprechender Berücksichtigung der unterschiedlichen Dosierung, den oben angegebenen Werten im wesentlichen.

Erfindungsgemäße Präparate ermöglichen eine mittlere Halbwertsdauer W₅₀ (bezogen wiederum auf eine einmalige Applikation von 100 mg Wirkstoff) von mehr als fünf Stunden, vorzugsweise von mehr als sechs Stunden und besonders bevorzugt von mehr als sieben Stunden.

Die erfindungsgemäßen pharmazeutischen Präparate enthalten wenigstens Tramadol und ihr Wirkstoffanteil enthält wenigstens Tramadol

Erfindungsgemäße pharmazeutische Präparate können also mehr als ein Opioid-Analgetikum umfassen, und sie können neben einem oder mehreren Opioid-Analgetika auch weitere Wirkstoffe umfassen. Andere Wirkstoffe, die mit Opioid-Analgetika kombiniert werden können, sind im Stand der Technik bekannt.

Die Analgetika, die in den erfindungsgemäßen Präparaten neben tramadol enthalten sein können, entsprechen den im eingangs schon genannten Stand der Technik angegebenen Wirkstoffen, insbesondere den in EP-A2 0 864 325 benannten Opioid-Analgetika.

Der analgetische Wirkstoff, ist vorzugsweise auf die beiden unterschiedlich freisetzenden "Phasen" des Präparates ungleich verteilt, so daß der relativ größere Teil des Wirkstoffes zur verzögerten Freisetzung formuliert ist. Generell ist also der Anteil des Analgetikums in der schnell freisetzenden Phase des Präparates kleiner als 50 %, und der Anteil des Wirkstoffes in der Retardmatrix größer als 50 %, bezogen auf den Gesamtgehalt an analgetischem Wirkstoff im Präparat. Besonders bevorzugt stehen die relativen Mengen des Wirkstoffes in der schnell freisetzenden bzw. der verzögert freisetzenden "Phase" der Tablette etwa im Verhältnis von 1 : 3 bis 1 : 5; bei einer erfindungsgemäßen Tablette mit insgesamt 100 mg Tramadol-Hydrochlorid entspricht das bei einem Verhältnis von 1 : 3 einer Verteilung von 25 mg Tramadol-Hydrochlorid im schnell freisetzenden Anteil und 75 mg Tramadol-Hydrochlorid im verzögert freisetzenden Anteil jeder Tablette.

Während die bevorzugte Einheitsdosis des Analgetikums, insbesondere für Tramadol-Hydrochlorid, bei etwa 100 mg liegt, kann sie hiervon abweichen. Der Wirkstoffgehalt eines erfindungsgemäßen Präparates liegt bei einer Einheitsdosis generell zwischen 20 und 500 mg, insbesondere zwischen 50 und 200 mg. Hohe Dosierungen werden weniger bevorzugt, da die Erfindung es ermöglicht, eine schnelle Anflutung mit einer lang anhaltenden Wirkungsdauer zu verbinden, ohne daß die maximale Wirkstoffkonzentration im Patientenblut unerwünscht hohe Werte erreicht.

Die erfindungsgemäßen Präparate sind vorzugsweise als feste Arzneiformen ausgebildet. Besonders bevorzugt werden Tabletten, insbesondere Zweischichttabletten. Der verzögert freisetzende Anteil des Analgetikums, vorzugsweise ein Opioid, wird dann in einer Schicht untergebracht, während der schnell freisetzende Anteil in der anderen Schicht aufgenommen wird. Außen ist die Tablette dann vorzugsweise mit einem Überzug versehen, der ihr insgesamt geschmacksneutrale Eigenschaften verleiht.

Der schnell freisetzende Teil des erfindungsgemäßen pharmazeutischen Präparates umfaßt ein potentes Sprengmittel, insbesondere gebildet aus einem Polymer. Besonders bevorzugt wird gegenwärtig quer-vernetztes PVP. Der Anteil des Sprengmittels im schnell freisetzenden Teil des Präparates muß so bemessen sein, daß die gewünschte schnelle Freisetzung und damit schnelle Anflutung des Wirkstoffes erreicht wird. Der Sprengmittelanteil entspricht daher wenigstens 10 Gew.-% der Gesamtmasse (einschließlich des Wirkstoffanteils) der schnell freisetzenden "Phase" des Präparates. Oft entspricht der Anteil wenigstens 20 Gew.-%, bevorzugt wenigstens 25 Gew.-% und besonders bevorzugt wenigstens 30 Gew.-%, wiederum bezogen auf die Masse des Teils des erfindungsgemäßen Präparates, die die schnell freisetzende Wirkstoff-Formulierung darstellt. Um eine besonders starke Anflutung und entsprechende anfängliche Wirkstoff-Freisetzung zu erreichen, kann der Sprengmittelanteil auch oberhalb 30 Gew.-% liegen; in einer besonders bevorzugten Ausführungsform der Erfindung liegt er bei etwa 32 Gew.-%, wiederum bezogen auf die gleiche Basis.

Eine solche erfindungsgemäße schnell freisetzende Formulierung enthält neben dem genannten Anteil an Sprengmittel, insbesondere quer-vernetztes PVP, noch den schnell freizusetzenden Anteil des Wirkstoffes, bei einer 100 mg Tramadol-Tablette also beispielsweise 25 mg Tramadol-Hydrochlorid. Weiterhin enthält die schnell freisetzende Formulierung vorzugsweise ein Lactose-Hydrat wie insbesondere Lactose-Monohydrat, dessen Anteil ebenfalls etwa 30 Gew.-%, bezogen auf die Gesamtmasse des schnell freisetzenden Teils des Präparates, erreichen kann.

Weitere Bestandteile, wie etwa Binder (vorzugsweise Polyvidon), Magnesium-Stearat und dergleichen können in üblichen Mengen zugesetzt werden. Der schnell freisetzende Anteil des Präparates enthält in der am meisten bevorzugten Ausführungsform daneben eine kleine Menge (etwa 1 mg) gereinigtes Wasser.

Die Retardmatrix, die beispielsweise in der zweiten Schicht einer erfindungsgemäßen Zweischichttablette vorgesehen wird, entspricht grundsätzlich den im Stand der Technik schon bekannten Retardmatrices für Opioid-Analgetika wie insbesondere Tramadol. Sie kann gemäß der schon genannten EP-A1 0 624 366 ausgebildet werden; die dort angegebenen Zusammensetzungen, sei es generell, sei es als Ausführungsbeispiel, lassen sich auch für die vorliegende Erfindung als Retardmatrix verwenden.

Beispielsweise enthält in einer gegenwärtig besonders bevorzugten Ausführungsform die zur verzögerten Freisetzung formulierte Zusammensetzung eines erfindungsgemäßen Präparates neben dem entsprechenden Anteil von Wirkstoff (bei einer 100 mg-Tablette also 75 mg Wirkstoff, insbesondere Tramadol-Hydrochlorid) ein Gemisch aus Lactose-Monohydrat, Ethylcellulose mit niedriger Viskosität und Cetostearylalkohol, als Hauptbestandteile. Daneben können weitere Formulierungshilfen eingesetzt werden, die im Stand der Technik bekannt sind.

Die Herstellung erfindungsgemäßer Präparate erfolgt gemäß im Stand der Technik bekannten und beschriebenen Verfahren. Beispielsweise lassen sich Tabletten im Sinne erfindungsgemäßer Präparate nach den Verfahren herstellen, die in der schon genannten EP-B 1 0 642 788 beschrieben sind.

Eine besonders wichtige Indikation für erfindungsgemäße Präparate ist die Akut-Schmerztherapie. Insbesondere wegen der schnellen und hohen Anflutung lassen sich solche Präparate hier besonders vorteilhaft statt der gegenwärtig üblichen Tropfen verwenden.

Im folgenden wird eine gegenwärtig besonders bevorzugte Ausführungsform der Erfindung beispielhaft beschrieben.

### Beispiel

Ein erfindungsgemäßes Präparat wird hergestellt, indem zunächst eine Retardmatrix im wesentlichen aus etwa 50 mg Lactose-Monohydrat, etwa 8 mg Ethylcellulose und etwa 32 mg Cetostearylalkohol, weiterhin etwa 2 mg Talkum, etwa 1,5 mg Magnesium-Stearat, etwa 1 mg Ölsäure, etwa 1,7 mg Dibutylsebacat und etwa 2 mg Wasser gebildet wird. In bekannter Weise werden 75 mg Tramadol-Hydrochlorid in diese Retardmatrix eingearbeitet. Die wirkstoffhaltige Retardmatrix wird als eine Schicht für eine Zweischichttablette eingesetzt.

Parallel wird aus 25 mg Tramadol-Hydrochlorid, 27,5 mg Lactose-Monohydrat, 1 mg Magnesium-Stearat, 4,25 mg Polyvidon, 27,5 mg mikrokristalline Cellulose und etwa 1 mg Wasser eine schnell freisetzende Formulierung erzeugt. Auch hier erfolgt die Zugabe und Vermischung der Bestandteile nach im Stand der Technik bekannten Verfahren.

Die die Retardmatrix mit der Hauptmasse des Wirkstoffes enthaltende Masse wird dann in bekannter Weise mit der wirkstoffhaltigen schnell freisetzenden Formulierung zu einer Zweischichttablette verarbeitet.

Die so gebildete Tablette wird dann mit einem Überzug versehen, der als Hauptbestandteil Hydroxypropylmethylcellulose, Polydextrose, Macrogol 4000 und Talkum enthält. Durch diesen Überzug ist die Tablette geschmacksneutral und läßt sich leichter schlucken.

Die Gesamtmasse der Tablette liegt bei etwa 290 mg, bei einem Gesamtgehalt von Tramadol-Hydrochlorid von 100 mg, der sich im Verhältnis 75 : 25 auf die Retardmatrixschicht und die schnell freisetzende Schicht der Tablette verteilt.

An einer Mehrzahl von Probanden wurden mit den im Beispiel beschriebenen Tabletten die Tramadol-Plasmaspiegelwerte in ihrer zeitlichen Entwicklung gemessen. An dem Versuch nahmen vierundzwanzig gesunde männliche Probanden teil. Der Test war als offener randomisierter Crossover-Test mit Einzeldosisgabe angelegt. Zwischen jeweils zwei Versuchen lag eine Pause (als "washout") von einer Woche.

Die Probanden erhielten zum Vergleich mit den im Beispiel angegebenen Tabletten die seit längerem im Handel erhältlichen Präparate Tramundin®, ein Kapselpräparat mit 50 mg Tramadol-Hydrochlorid und schneller Freisetzung, und Tramundin® retard mit 100 mg Tramadol-Hydrochlorid in einer Retardformulierung, etwa entsprechend EP-A1 0 624 366.

Die Blutentnahme zur Bestimmung der Plasmawerte erfolgte vor der Verabreichung und dann in den zeitlichen Abständen, die der folgenden Tabelle 1 entnehmbar sind.

**Tabelle 1**

| Zeit [h] | Plasmaspiegel [ng/ml] | | |
|---|---|---|---|
| | Tramundin SL | Tramundin | Tramundin retard |
| 0,00 | 0,00 | 0,00 | 0,00 |
| 0,17 | 4,98 | 1,57 | 1,32 |
| 0,33 | 16,44 | 7,05 | 7,82 |
| 0,50 | 46,02 | 19,20 | 15,38 |
| 0,75 | 69,11 | 40,03 | 30,04 |
| 1,00 | 87,53 | 54,57 | 45,95 |
| 1,25 | 97,97 | 74,74 | 67,78 |
| 1,50 | 110,53 | 89,44 | 85,57 |
| 2,00 | 109,27 | 103,14 | 96,58 |
| 2,50 | 106,58 | 97,78 | 108,10 |
| 3,00 | 100,65 | 83,76 | 107,77 |
| | | | |
| 4,00 | 92,25 | 69,84 | 106,18 |
| 5,00 | 85,54 | 61,28 | 95,37 |
| 6,00 | 74,02 | 48,41 | 86,21 |
| 8,00 | 64,22 | 41,22 | 77,78 |
| 10,00 | 52,40 | 31,56 | 68,32 |
| 12,00 | 40,22 | 24,62 | 53,48 |
| | | | |
| | | | |
| 18,00 | 29,83 | 14,32 | 37,77 |
| | | | |
| 24,00 | 14,75 | 7,40 | 18,32 |
| | | | |
| 36,00 | 4,80 | 1,63 | 6,76 |
| | | | |

| | | | |
|---|---|---|---|
| "Tramundin SL" ist die im Beispiel beschriebene zweiphasige Tablette gemäß der Erfindung. | | | |

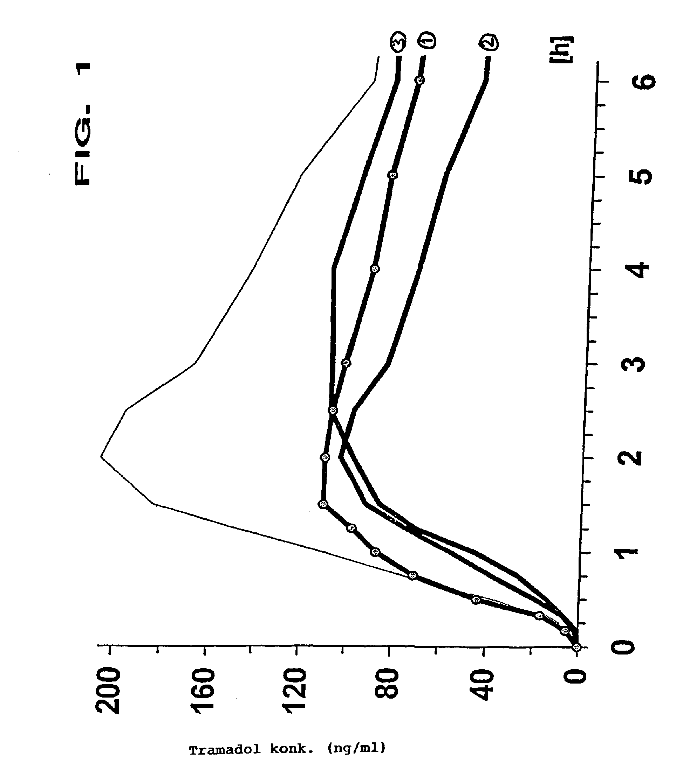

Aus der Entwicklung der Plasmaspiegel läßt sich die im Vergleich mit den bekannten Tramadol-Formulierungen deutlich schnellere anfängliche Anflutung genauso erkennen wie die lang anhaltende Aufrechterhaltung relevanter Plasmaspiegel. In der beigefügten Figur 1 ist dies graphisch dargestellt, wobei die mit 1 bezeichnete Kurve dem erfindungsgemäßen "zweiphasigen" Präparat Tramundin SL entspricht, das schon im obigen Beispiel beschrieben wurde; die mit 2 bezeichnete Kurve entspricht dem Präparat Tramundin, also der schnell freisetzenden Kapsel, und die mit 3 bezeichnete Kurve umfaßt die Werte für Tramundin retard, wie oben angegeben. Hinzu kommt eine dünn eingezeichnete Kurve, die einer Umrechnung der Werte der Kurve Nr. 2 auf einen Wirkstoffgehalt von 100 mg Tramadol-Hydrochlorid entspricht.

Wie schon den angegebenen Zahlenwerten kann auch der Figur 1 unmittelbar entnommen werden, daß das erfindungsgemäße Präparat eine sehr schnelle Anflutung zeigt, wie sie sonst nur bei einem insgesamt schnell freisetzenden Präparat erhältlich wäre, das aber zu sehr hohen Cₘₐₓ-Werten führen würde, und dies mit einer sehr lang anhaltenden Wirkung vereinigt, die nicht retardierten Formulierungen deutlich überlegen ist und die Werte der nur retardierten bekannten Formulierung Tramundin retard fast erreicht. Es wird also eine gewünschte schnelle Anflutung verbunden mit einer sehr großen Halbwertsdauer, ohne daß der Patient zwischen Eintritt und Nachlassen der Wirkung unnötig hohen Wirkstoffkonzentrationen ausgesetzt wird.

## Patentansprüche

1. Pharmazeutisches Präparat zur oralen Verabreichung, mit wenigstens einem Wirkstoff und mit die Wirkstoff-Freisetzung beeinflussenden Formulierungsbestandteilen, das als Wirkstoff wenigstens Tramadol oder seine physiologisch verträglichen Salze sowie Derivate umfasst, welches anteilig einerseits zur schnellen Freisetzung, andererseits zur verzögerten Freisetzung so formuliert ist, dass
die *in vitro* Freisetzungsrate aus dem Präparat gemäß dem Ph. Eur.-Paddeltest im Mittelwert nach einer Stunde bei oberhalb 40 Gew.-% und nach vier Stunden noch unter 80 Gew.-% Wirkstoff liegt, und
das Präparat den zur verzögerten Freisetzung formulierten Anteil des Wirkstoffes in einer Retardmatrix enthält, die eine oder mehrere Alkylcellulosen gemeinsam mit einem oder mehreren aliphatischen C₁₂-C₃₆ Alkohol umfasst.

2. Präparat nach Anspruch 1,
**dadurch gekennzeichnet, dass** die Freisetzungsrate nach vier Stunden bei über 70 Gew.-% liegt.

3. Präparat nach Anspruch 1 oder 2,
**dadurch gekennzeichnet, dass** die Freisetzungsrate nach vierzehn Stunden, insbesondere nach sechzehn Stunden noch unter 100 Gew.-% liegt und vorzugsweise nach sieben bis zehn Stunden, besonders bevorzugt nach etwa acht Stunden, 90 Gew.-% erreicht.

4. Pharmazeutisches Präparat, nach Anspruch 1
**dadurch gekennzeichnet, dass** der Wirkstoff-Plasmaspiegel im Mittel folgende zeitliche Entwicklung zeigt:
| | | | | | | |
|---|---|---|---|---|---|---|
| Mindestens | 2 ng/ml | Vorzugsweise mindestens | 3 ng/ml | Besonders bevorzugt mindestens | 4 ng/ml | nach 0,15 h |
| " | 10 ng/ml | " | 13 ng/ml | " | 15 ng/ml | nach 0,30 h |
| " | 35 ng/ml | " | 40 ng/ml | " | 45 ng/ml | nach 0,50 h |
| " | 55 ng/ml | " | 60 ng/ml | " | 65 ng/ml | nach 0,75 h |
| " | 75 ng/ml | " | 80 ng/ml | " | 85 ng/ml | nach 1,00 h |
| " | 85 ng/ml | " | 90 ng/ml | " | 95 ng/ml | nach 1,25 h |
| " | 100 ng/ml | " | 105 ng/ml | " | 110 ng/ml | nach 1,50 h |
auf Basis einer einmaligen Verabreichung von 100 mg des Analgetikums, oder eine entsprechende zeitliche Entwicklung auf der Basis einer anderen Einmaldosis des Analgetikums zeigt.

5. Pharmazeutisches Präparat nach Anspruch 4,
**dadurch gekennzeichnet, dass** der Wirkstoff-Plasmaspiegel im Mittel folgende zeitliche Entwicklung zeigt:
| | |
|---|---|
| Plasmakonzentration | Zeitraum ab Verabreichung |
| zwischen 90 und 120 ng/ml | zwischen 2 h und 4 h; |
| zwischen 60 und 100 ng/ml | zwischen 4 h und 8 h; |
| zwischen 40 und 60 ng/ml | zwischen 8 h und 11 h und |
insbesondere oberhalb 30 ng/ml bis 16 h nach Verabreichung,
auf Basis einer einmaligen Verabreichung von 100 mg des Analgetikums, oder eine entsprechende zeitliche Entwicklung auf der Basis einer anderen Einmaldosis des Analgetikums zeigt.

6. Präparat nach einem der Ansprüche 1 bis 5,
**dadurch gekennzeichnet, dass** das Präparat Tramadol-Hydrochlorid enthält, wobei vorzugsweise weniger als 50 % des Wirkstoffs zur schnellen Freisetzung formuliert ist und besonders bevorzugt der Wirkstoff etwa im Verhältnis 3 : 1 bis 5 : 1 auf die verzögert freisetzende Phase und die schnell freisetzende Phase des Präparates verteilt ist.

7. Präparat nach einem der Ansprüche 1 bis 6,
**dadurch gekennzeichnet, dass** das Präparat insgesamt zwischen 20 und 500 mg, insbesondere zwischen 50 und 200 mg und besonders bevorzugt etwa 100 mg Tramadol-Hydrochlorid pro Einheitsdosis enthält.

8. Präparat nach einem der Ansprüche 1 bis 7,
**dadurch gekennzeichnet, dass** der schnell freisetzende Wirkstoffanteil mit einem Sprengmittel, insbesondere einem Polymer und besonders bevorzugt mit quervemetztem Polyvinylpyrrolidon formuliert ist.

9. Präparat nach Anspruch 8,
**dadurch gekennzeichnet, dass** der Sprengmittelanteil der schnell freisetzenden Formulierung wenigstens 0,5-10 Gew.-%, vorzugsweise 1 - 5 Gew.-%, und besonders bevorzugt 3 - 4 Gew.-%, bezogen auf den Gewichtsanteil der schnell freisetzenden Formulierung, ausmacht.

10. Präparat nach einem der Ansprüche 1 bis 9,
wobei es sich bei der Alkylcellulose um Ethylcellulose und bei dem aliphatischen Alkohol um Laurylalkohol, Myristylalkohol, Stearylalkohol, Cetylalkohol oder Cetostearylalkohol handelt.

11. Präparat nach einem der Ansprüche 1 bis 10,
**dadurch gekennzeichnet, dass** die Alkylcellulose der Retardierungsmatrix in 2 Gew.-%iger wässriger Lösung bei 20°C eine Viskosität von weniger als 3000 mPas, vorzugsweise weniger als 2000, besonders bevorzugt weniger als 1000 und speziell weniger als 500 mPas aufweist.

12. Präparat nach Anspruch 11,
**dadurch gekennzeichnet, dass** das Präparat als Zweischichttablette so ausgebildet ist, dass der verzögert freisetzende Anteil in einer Tablettenschicht und der schnell freisetzende Anteil in der anderen Tablettenschicht vorgesehen ist.

13. Präparat nach einem der vorstehenden Ansprüche,
**dadurch gekennzeichnet, dass** das Präparat als Tablette ausgebildet und mit einem geschmacksneutralen Überzug versehen ist.

## Claims

1. Pharmaceutical preparation, especially for oral administration, with at least one active agent and with formulation components which influence the release of said active agent, which comprises tramadol or its pharmaceutically acceptable salts as an active agent, and which is formulated partly for immediate release, partly for sustained release in such a way that the average *in vitro* release rate from the preparation, in accordance with the Ph. Eur. paddle method, is above 40 weight-% after one hour,
and below 80 weight-% after four hours and wherein the preparation comprises the sustained release portion of the active in a matrix that comprises at least one alkyl-cellulose and at least one aliphatic C₁₂-C₃₆ alcohol.

2. Preparation according to claim 1,
**characterized in that** the release rate after four hours is above 70 weight-%.

3. Preparation according to claim 1 or 2,
**characterized in that** the release rate after fourteen hours, especially after sixteen hours, is still below 100 weight-% and preferably reaches 90 weight-% after seven to ten hours, especially preferably after approximately eight hours.

4. Pharmaceutical preparation according to claim 1,
**characterized in that** the average plasma levels of active agent show the following development with time:
| | | | | | | |
|---|---|---|---|---|---|---|
| At least | 2 ng/ml | Preferably at least | 3 ng/ml | Especially preferred at least | 4 ng/ml | after 0,15 h |
| " | 10 nglml | " | 13 ng/ml | " | 15 ng/ml | after 0,30 h |
| " | 35 ng/ml | " | 40 ng/ml | " | 45 ng/ml | after 0,50 h |
| " | 55 ng/ml | " | 60 ng/ml | " | 65 ng/ml | after 0,75 h |
| " | 75 ng/ml | " | 80 ng/ml | " | 85 ng/ml | after 1,00 h |
| " | 85 ng/ml | " | 90 ng/ml | " | 95 ng/ml | after 1,25 h |
| " | 100 ng/ml | " | 105 ng/ml | " | 110 ng/ml | after 1,50 h |
based on a single application of 100 mg analgesc, or show a corresponding development over time for another single dose of analgesc.

5. Pharmaceutical preparation according to claim 4,
**characterized in that** the average plasma levels of active agent show the following development with time:
| | |
|---|---|
| Plasma concentration | Time after application |
| between 90 and 120 ng/ml | between 2 h and 4 h; |
| between 60 and 100 ng/ml | between 4 h and 8 h; |
| between 40 and 60 ng/ml | between 8 h and 11 h and |
based on single application of 100 mg of analgesc, or show a corresponding development over time for another single dose of analgesc.

6. Preparation according to any of claims 1 to 5,
**characterized in that** the preparation comprises tramadol-hydrochloride, whereby preferably, less than 50 % of the active agent are formulated for immediate release and especially preferably, the agent is distributed approximately in a 3 : 1 to 5 : 1 ratio in the sustained release phase and the fast release phase, respectively, of the preparation.

7. Preparation according to any of claims 1 to 6,
**characterized in that** the preparation contains, in total, between 20 and 500 mg, especially between 50 and 200 mg and especially preferably approximately 100 mg tramadol-hydrochloride per unit dosis.

8. Preparation according to any of claims 1 to 7,
**characterized in that** the immediate release part of the active agent is formulated with a bursting agent, preferably a polymer and especially preferred with cross-linked polyvinylpyrrolidone.

9. Preparation according to any of claims 1 to 8,
**characterized in that** the amount of bursting agent in the immediate releasing formulation is at least 0.5 to 10 % by weight, preferably 1 to 5 % by weight and especially preferred 3 to 4 % by weight of the immediate releasing portion of the preparation.

10. Preparation according to any of claims 1 to 9,
**characterized in that** the alkyl cellulose is ethyl cellulose, and the aliphatic alcohol is lauryl alcohol, myristyl alcohol, stearyl alcohol, cetyl alcohol or cetostearyl alcohol.

11. Preparation according to any of claims 1 to 10,
**characterized in that** the alkyl cellulose of the sustained release matrix has a viscosity of less than 3000 mPas, preferably less than 2000, especially preferably less than 1000 and especially less than 500 mPas, in 2 weight-% aqueous solution, at 20°C.

12. Preparation according to claim 11,
**characterized in that** the preparation is embodied as a two-layer tablet such that the sustained release phase is provided in one tablet layer and the fast release phase is provided in the second tablet layer.

13. Preparation in accordance with one of the preceeding claims,
**characterized in that** the preparation is embodied as a tablet and provided with a taste neutral coating.

## Revendications

1. Préparation pharmaceutique pour administration orale, avec au moins un agent actif et avec des composants de formulation influençant la libération de l'agent actif qui comprennent comme agent actif au minimum le tramadol ou ses sels ou dérivés physiologiquement compatibles, et qui est formulée d'une part pour une libération rapide, d'autre part pour une libération retardée, proportionnellement de telle sorte que
- le taux de libération *in vitro* à partir de la préparation après une heure suivant le test Ph. Eur. Paddletest soit en moyenne supérieur à 40 % en poids et après quatre heures reste encore inférieur à 80 % en poids de substance active, et
- la préparation de la portion formulée pour libération retardée de la substance active soit contenue dans une matrice retard qui comprend une ou plusieurs alkylcelluloses associées à un ou plusieurs alcools aliphatiques C₁₂-C₃₆.

2. Préparation selon la revendication 1 **caractérisée en ce que** le taux de libération est supérieur à 70 % en poids après quatre heures.

3. Préparation selon la revendication 1 ou 2 **caractérisée en ce que** le taux de libération après quatorze heures, en particulier après seize heures, est encore inférieur à 100 % en poids, et de préférence après sept à dix heures, tout particulièrement après environ huit heures, atteint 90 % en poids.

4. Préparation pharmaceutique selon la revendication 1 **caractérisée en ce que** le niveau de substance active dans le plasma au milieu du développement chronologique suivant affiche :
Minimum 2 ng/ml, de préférence min. 3 ng/ml,
tout particulièrement min. 4 ng/ml après 0,15 h
Minimum 10 ng/ml, de préférence min. 13 ng/ml,
tout particulièrement min. 15 ng/ml après 0,30 h
Minimum 35 ng/ml, de préférence min. 40 ng/ml,
tout particulièrement min. 45 ng/ml après 0,50 h
Minimum 55 ng/ml, de préférence min. 60 ng/ml,
tout particulièrement min. 65 ng/ml après 0,75 h
Minimum 75 ng/ml, de préférence min. 80 ng/ml,
tout particulièrement min. 85 ng/ml après 1,00 h
Minimum 85 ng/ml, de préférence min. 90 ng/ml,
tout particulièrement min. 95 ng/ml après 1,25 h
Minimum 100 ng/ml, de préférence min. 105 ng/ml,
tout particulièrement min. 110 ng/ml après 1,50 h,
sur la base d'une administration en une fois de 100 mg de l'analgésique, ou d'un développement chronologique correspondant sur la base d'une autre dose unique de l'analgésique.

5. Préparation pharmaceutique selon la revendication 4 **caractérisée en ce que** le niveau de substance active dans le plasma au milieu du développement chronologique suivant affiche :
| | |
|---|---|
| Concentration dans le plasma | Temps après administration |
| entre 90 et 120 ng/ml | entre 2 et 4 h |
| entre 60 et 100 ng/ml | entre 4 et 8 h |
| entre 40 et 60 ng/ml | entre 8 et 11 h, |
en particulier supérieure à 30 ng/ml jusqu'à 16 heures après administration, sur la base d'une administration en une fois de 100 mg de l'analgésique, ou d'un développement chronologique correspondant sur la base d'une autre dose unique de l'analgésique.

6. Préparation selon une des revendications 1 à 5 **caractérisée en ce que** la préparation contient du chlorhydrate de tramadol, où de préférence moins de 50 % de la préparation sont formulés pour la libération rapide et tout particulièrement l'agent actif est dans un rapport d'environ 3:1 à 5:1 par rapport à la phase à libération retardée, et la phase à libération rapide de la préparation est répartie.

7. Préparation selon une des revendications 1 à 6 **caractérisée en ce que** la préparation contient en tout entre 20 et 500 mg, de préférence entre 50 et 200 mg, et tout particulièrement environ 100 mg de chlorhydrate de tramadol par dose unitaire.

8. Préparation selon une des revendications 1 à 7 **caractérisée en ce que** la portion d'agent actif à libération rapide est formulée avec une substance à décomposition rapide, en particulier un polymère et tout particulièrement de la polyvinylpyrrolidone à chaînes latérales.

9. Préparation selon la revendication 8 **caractérisée en ce que** la portion en substance à décomposition rapide de la formulation à libération rapide représente au moins 0,5-10 % en poids, de préférence 1-5 % en poids et tout particulièrement 3-4 % en poids rapportés à la portion massique de la formulation à libération rapide.

10. Préparation selon une des revendications 1 à 9 où pour ce qui est de l'alkylcellulose, il s'agit d'éthylcellulose, et pour ce qui est de l'alcool aliphatique, il s'agit d'alcool laurylique, myristique, stéarique, cétylique ou cétostéarique.

11. Préparation selon une des revendications 1 à 10 **caractérisée en ce que** l'alkylcellulose présente dans une solution aqueuse à 2 % en poids à 20°C une viscosité inférieure à 3000 mPa.s, de préférence inférieure à 2000, tout particulièrement inférieure à 1000 et spécialement inférieure à 500 mPa.s.

12. Préparation selon la revendication 11 **caractérisée en ce que** la préparation est développée sous forme de comprimés à deux couches, de sorte que la portion à libération retardée soit prévue dans une couche du comprimé et la portion à libération rapide soit prévue dans l'autre couche du comprimé.

13. Préparation selon une des revendications précédentes **caractérisée en ce que** la préparation est développée sous forme de comprimés et est pourvue d'une couche de couverture sans goût.
